# FASCICULE DE BREVET EUROPEEN

(11) **EP 0 494 806 B1**
(45) Date de publication et mention de la délivrance du brevet: **05.04.1995**
(21) Numéro de dépôt: 92400015.1
(22) Date de dépôt: 06.01.1992
(51) Int. Cl.: A61M 25/06, A61M 25/00

(54) **Cathéter multilumière intravasculaire, susceptible d'être implanté avec tunnellisation**
Intravaskulärer mehrlumiger Katheter für das Implantieren mit Hilfe eines Tunnels
Intravascular multilumen catheter for tunneling implantation

(30) Priorité: 08.01.1991 FR 9100139
(43) Date de publication de la demande: 15.07.1992
(73) Titulaire: Durand, Alain, Jean-Marie, F-3000 Nimes (FR)
(72) Inventeur: Durand, Alain, Jean-Marie, F-3000 Nimes (FR)
(74) Mandataire: Boutin, Antoine

(56) Documents cités:
- EP-A- 0 090 687
- EP-A- 0 263 645
- US-A- 4 453 928
- US-A- 4 619 643

## Description

La présente invention concerne un instrument médical du genre cathéter dont la tubulure comporte au moins deux lumières.

Le terme "médical" est pris içi dans son sens le plus général, et concerne aussi bien les applications relevant de la médecine humaine que de l'art vétérinaire, tandis que la désignation de "cathéter intravasculaire" vise un dispositif devant être placé dans le système vasculaire d'un patient, par ponction du vaisseau choisi.

Lorsqu'un cathéter est destiné à un usage prolongé sur un patient, ce cathéter est mis en place selon la technique de SELDINGER, consistant à ponctionner un vaisseau avec une aiguille de faible diamètre, dont la lumière permet le passage d'un fin guide spiralé, à retirer l'aiguille de ponction lorsque le guide est suffisamment introduit dans le vaisseau, puis à faire progresser la tubulure du cathéter sur le guide, enfin à retirer le guide en laissant le cathéter en place dans le vaisseau. Cependant en général on procède également à la "tunnellisation" de la tubulure du cathéter sur une certaine longueur. Ceci consiste à faire suivre à la tubulure du cathéter, un cheminement de plusieurs centimètres entre le point de ponction du vaisseau et la surface externe du corps du patient, et ce à peu près parallèlement à la peau de celui-ci et à quelques millimètres de sa surface. Ainsi tous les germes extérieurs, susceptibles de progresser le long de la tubulure du cathéter vers le vaisseau ponctionné, ou vers un organe du patient, se heurtent à l'obstacle naturel formé par les tissus traversés lors de la réalisation de la "tunnellisation".

Un cathéter spécialement conçu pour l'application de cette technique est décrit dans le brevet FR-A-2.522.504. Cependant il s'agit d'un cathéter monolumière.

Or, le développement des techniques diagnostiques et thérapeutiques a montré la nécessité de disposer de cathéters constitués par une tubulure multilumière, de façon à pouvoir utiliser plusieurs lumières distinctes, soit pour des fonctions identiques et néanmoins nécessairement indépendantes, par exemple dans le cas de perfusion simultanée de deux médicaments incompatibles entre eux à l'état pur, Un tel cathéter est connu du EP-A-0 263 645. D'autres cathéters existent qui permettent par exemple monitoring de pression intravasculaire, de la concentration de C02, perfusion, prélèvement de sang, etc.

Avec le matériel existant, il s'avère cependant très difficile, pour ne pas dire impossible, pour un praticien de pouvoir appliquer la pratique de la "tunnellisation" à un cathéter multilumière qui présente inévitablement une section plus importante qu'un cathéter monolumière. De plus, il faut alors réaliser, dans la chair du patient, un passage de diamètre largement supérieur au diamètre extérieur du cathéter, ce qui revient à annihiler, ou tout au moins à réduire, le rôle de barrière aux agressions extérieures justement recherché par la pratique de la tunnellisation.

C'est pourquoi la présente invention a pour but de réaliser un dispositif de cathéter à plusieurs lumières qui peut néanmoins faire l'objet d'une tunnellisation sans rencontrer les inconvénients exposés ci-dessus.

A cet effet, la tubulure multilumière de ce cathéter constitue seulement la partie distale de celui-ci et ce dernier comprend en outre :
- des tubes distincts en nombre égal aux lumières de la première tubulure et qui s'étendent en amont de celle-ci pour former la partie intermédiaire du cathéter,
- une tubulure monolumière constituant l'extrêmité proximale de l'ensemble et qui porte en bout une aiguille rigide apte à permettre la réalisation de la tunnellisation,
- deux raccords assurant respectivement la jonction de chaque lumière de la tubulure multilumière avec un des tubes distincts de la partie intermédiaire, et le raccordement de l'extrêmité opposée de ces tubes avec l'extrêmité correspondante de la tubulure monolumière, caracterise en ce que chacun de ces raccords est constitué par un corps en forme d'olive effilée, surmoulé en matière plastique sur les extrêmités des parties à réunir.

Grâce à la subdivision de ce cathéter en plusieurs parties de natures distinctes et grâce à la jonction de ces dernières par l'intermédiaire de raccords surmoulés de forme effilée, il est possible de pratiquer sans difficulté la technique de la tunnellisation. Après application de cette technique, seule la tubulure distale multilumière reste implantée dans le corps du patient, de sorte qu'il suffit de couper le cathéter dans sa partie intermédiaire pour disposer de deux ou plusieurs tubes indépendants sur lesquels il est aisé de brancher les raccords amovibles nécessaires.

Cependant la présente invention a également pour objet un procédé particulier de fabrication d'un dispositif de cathéter tel que défini ci-dessus. Du reste, d'autres particularités et avantages de l'objet de l'invention apparaitront au cours de la description suivante. Celle-ci est donné en référence au dessin annexé à simple titre indicatif, et sur lequel :
La figure 1 est une vue en plan de dessus d'un cathéter selon l'invention.
Les figures 2 et 3 en sont des vues partielles en coupe longitudinale, respectivement à l'endroit de l'un et l'autre des deux raccords prévus sur ce cathéter.
Les figures 4 à 7 sont des vues en coupe transversale selon les lignes IV-IV, V-V, VI-VI et VII-VII de la figure 2, mais à échelle différente.
Les figures 8 à 11 sont des vues en coupe transversale selon les lignes VIII-VIII, IX-IX, X-X et XI-XI de la figure 3, mais à échelle différente.
La figure 12 est une vue schématique en coupe du moule utilisé pour la réalisation du raccord représenté à la figure 2.
La figure 13 est une vue en plan de l'aiguille de ponction utilisée pour la mise en place du présent cathéter.
Les figures 14 à 20 sont des schémas de différentes phases successives de l'opération de tunnellisation.
La figure 21 est une vue en plan de dessus de l'un des raccords amovibles rapportés ultérieurement sur l'un des tubes de la partie intermédiaire du présent cathéter après la tunnellisation de celui-ci.
La figure 22 est une vue en coupe longitudinale d'une autre forme de réalisation du raccord représenté à la figure 2.
Les figures 23 à 25 sont des vues en coupe transversale selon les lignes XXIII-XXIII, XXIV-XXIV et XXV-XXV de la figure 22.
La figure 26 est une vue en coupe transversale d'une autre forme de réalisation encore du raccord considéré, et ce dans le cas d'un cathéter dont la partie distale est constituée par une tubulure à trois lumières.

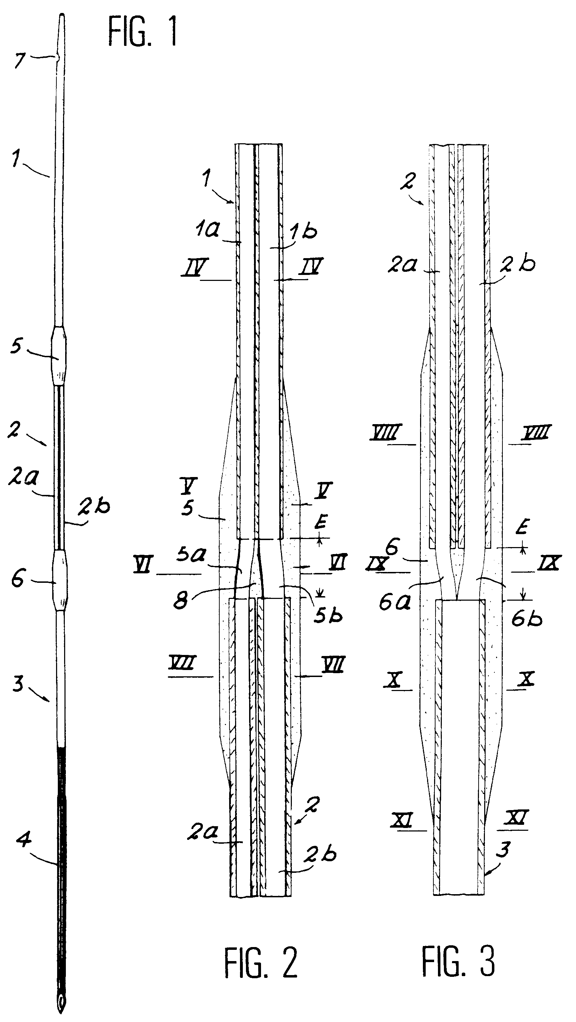

Comme il a déjà été indiqué, le cathéter représenté aux figures 1 à 12 comprend trois parties successives de structures différentes, à savoir :
- une partie distale désignée par la référence générale 1 et qui est constituée par une tubulure multilumière, en l'occurrence dans l'exemple représenté une tubulure comportant deux lumières 1a et 1b,
- une partie intermédiaire désignée par la référence générale 2 et qui est constituée par des tubes distincts 2a et 2b, en nombre égal à ceux des lumières la et 1b de la partie distale,
- une partie proximale désignée par la référence générale 3 et qui est constituée par une tubulure monolumière portant en bout une aiguille rigide 4 apte à permettre la réalisation de la tunnellisation.

Quant à l'extrémité libre de la partie distale 1, elle présente pour particularité qu'une seule des lumières de la tubulure 1 débouche à cet endroit, afin que celle-ci puisse servir au coulissement de l'ensemble sur le guide spiralé utilisé pour la mise en place du cathéter. L'autre lumière de la partie distale 1 débouche à travers un petit orifice 7 prévu sur le côté, à faible distance de l'extrémité.

Ces trois parties successives 1, 2 et 3 sont réunies par l'intermédiaire de deux raccords 5 et 6 constitués chacun par un corps en matière plastique, présentant la forme d'une olive effilée, et qui est surmoulé sur les extrémités des parties à réunir.

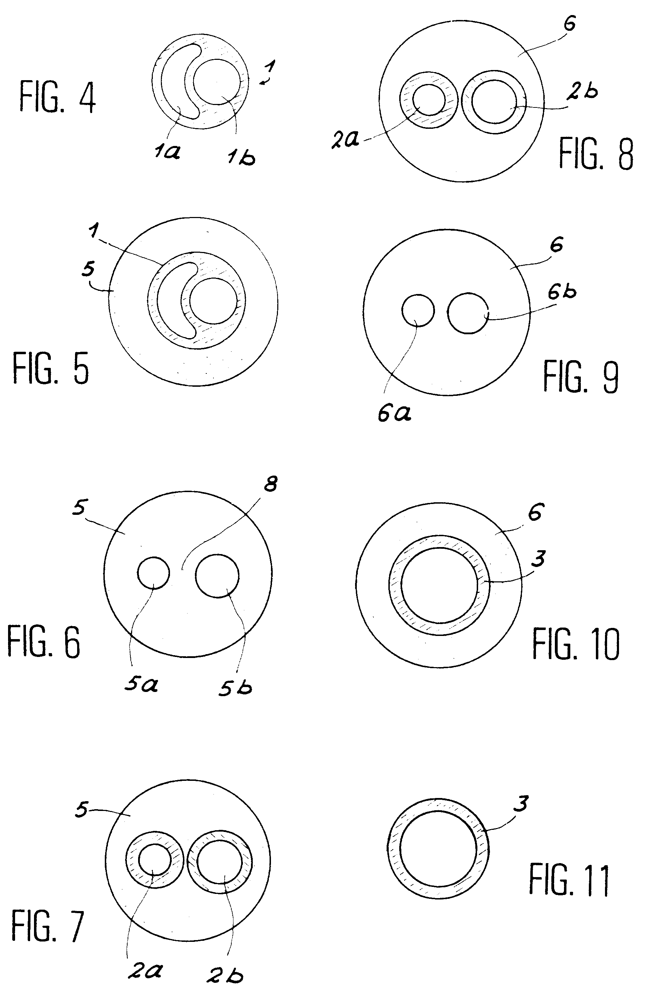

La figure 2, ainsi que les figures 4 à 7 représentent le premier raccord 3 qui réunit la partie distale 1 avec la partie intermédiaire 2.

Outre la forme externe particulière de ce raccord, une autre caractéristique essentielle de celui-ci réside dans le fait qu'il existe un écart E entre les extrémités des tubulures raccordées par ce raccord. La partie correspondante du corps de celui-ci comporte des canaux 5a et 5b ménagés à l'intérieur de la matière moulée et qui présentent entre eux un écart dans le sens transversal (voir figure 6). Ainsi il existe en quelque sorte un pont de jonction 8 entre les deux parties surmoulées de part et d'autre du plan passant par les axes des canaux 5a et 5b. Ceci assure une parfaite solidarisation du corps du raccord 5 avec les tubulures correspondantes en empêchant notamment toute possibilité de glissement dans le sens longitudinal.

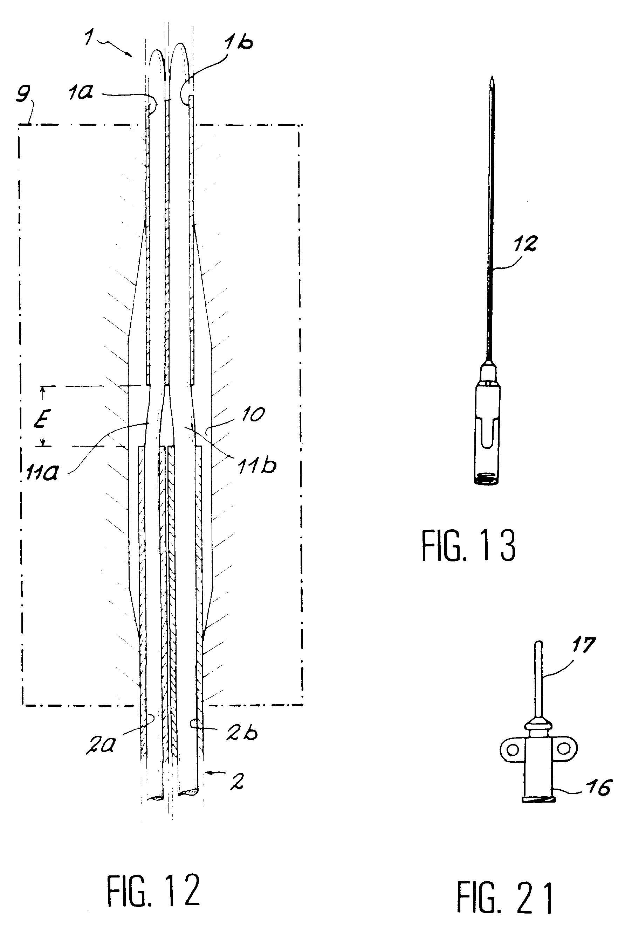

La figure 12 représente le moule utilisé pour la réalisation de ce raccord. Avant mise en place des extrémités des tubulures 1 et 2 à l'intérieur de la cavité de moulage 10 de ce moule, deux mandrins 11a et 11b sont enfilés à l'intérieur de ces tubulures. En l'occurrence le premier mandrin 11a est enfilé à la fois dans la lumière 1a de la tubulure 1 et à l'intérieur de l'un des tubes 2a de la partie intermédiaire 2 du cathéter. Bien entendu, l'autre mandrin est enfilé à la fois à l'intérieur de la lumière 1b de la tubulure 1 et à l'intérieur du tube 2b de la partie intermédiaire 2. Mais l'écart E est alors respecté entre les extrémités correspondantes des tubulures à réunir. Ainsi les deux mandrins 11a et 11b assurent la formation des canaux de liaison 5a et 5b à l'intérieur de la partie correspondante du corps du raccord 5.

Il faut noter que dans cette forme de réalisation ce raccord forme un manchon à la fois autour de l'extrémité correspondante de la tubulure 1 à deux lumières et autour des extrémités voisines des deux tubes 2a et 2b de la partie intermédiaire du cathéter. Toutefois l'épaisseur des parois de ce manchon est réduite au minimum techniquement possible, afin que la section transversale du raccord 5 soit elle-même réduite pour faciliter la tunnellisation ultérieure du cathéter.

Pour assurer la sécurité de l'ensemble après surmoulage, la partie correspondante du cathéter est ensuite placée entre deux électrodes de soudure dont la partie active a la même forme que le moule de surmoulage. Puis on applique un courant à haute fréquence qui fait fondre, ou tout au moins ramollit suffisamment l'ensemble pour qu'il y ait formation d'une pièce monobloc. A cet effet, il convient de choisir, pour la constitution des différentes pièces, des matières plastiques de même famille. Par exemple, dans le cas du choix de polyuréthane aliphatique pour la constitution des tubulures, la matière de surmoulage des raccords doit être également un polyuréthane aliphatique, les duretés de ces polyuréthanes étant, si possible, très proches, ce qui entraine également une similitude des températures de fusion.

Le raccord 6 qui réunit la partie intermédiaire 2 à la partie proximale 4 est réalisé de la même façon que le raccord 5 et avec un moule similaire. Là encore il est prévu un écart E entre les extrémités des tubulures à réunir, et ce dans le même but que précédemment. A cet endroit le corps du raccord 6 comporte donc des canaux 6a et 6b qui sont écartés l'un de l'autre et qui assurent chacun le raccordement d'un tube 2a ou 2b avec la lumière unique de la tubulure 3.

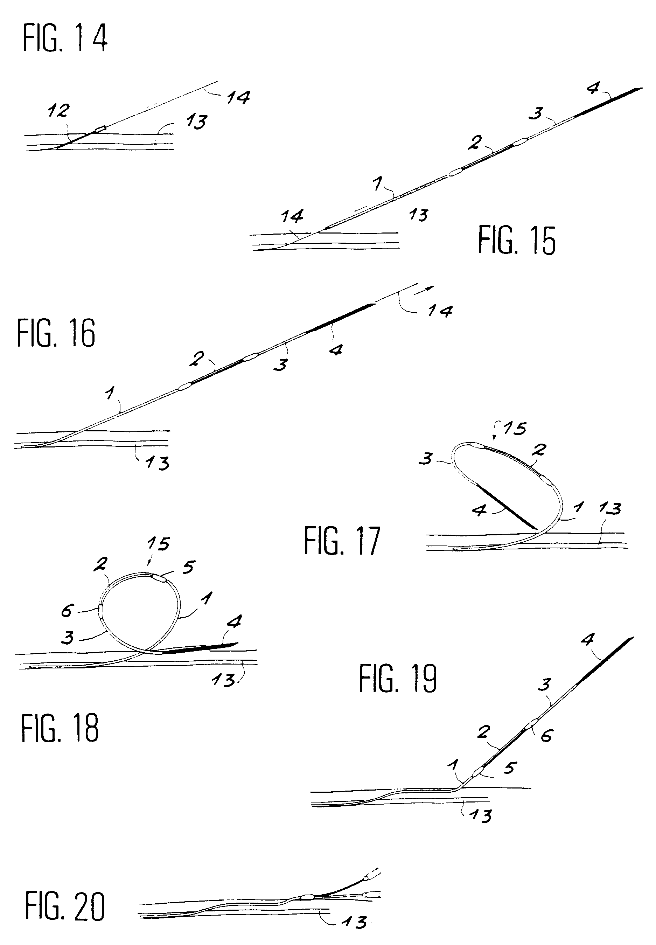

Les schémas des figures 14 à 20 illustrent les différentes phases successives de l'opération de mise en place du présent cathéter avec tunnellisation de celui-ci :
A - Après désinfection habituelle, le praticien ponctionne le tissu qu'il a choisi de cathétériser à l'aide d'une aiguille de ponction 12 d'une longueur de 8cm environ, ce qui permet d'atteindre facilement le vaisseau choisi 13, par exemple la veine sous clavière ou la veine fémorale, et ce même sur un sujet obèse. En général, le praticien ne manipule pas directement cette aiguille, mais commence par la fixer sur une seringue munie d'un embout normalisé ISO du type luer ou luer lock. Une fois obtenu un bon reflux du sang, le praticien retire la seringue et introduit, dans l'aiguille 12, un guide métallique spiralé 14 sur une quinzaine de centimètres environ (voir figure 14).
B - Maintenant l'extrémité du guide par pression sur la veine au delà de la pointe de l'aiguille de ponction 12, le praticien retire cette aiguille de ponction et la jette. Le praticien saisit alors d'une main l'extrémité du guide spiralé 14 restée extérieure au patient et y adapte l'extrémité distale du cathéter 1-2-3 selon l'invention. Puis il fait progresser entièrement ce cathéter sur le guide 14 jusqu'à ce qu'il puisse pénétrer dans le vaisseau choisi 13, ce cheminement étant toujours celui du guide 14 préalablement mis en place (voir figure 15).
C - Une fois ce cathéter suffisamment avancé, le guide spiralé 14 dépasse de l'extrémité de l'aiguille 4. Le praticien peut alors saisir ce guide et le retirer doucement sans modifier la position de l'extrémité du cathéter situé dans le vaisseau 13 (voir figure 16).
D - A ce moment le praticien saisit l'aiguille de tunnellisation 4 et pique exactement dans le point de ponction initial en inclinant latéralement le cathéter (figure 17). Pour faciliter la suite de l'opération il peut donner alors un léger coup de bistouri afin d'élargir le lieu de croisement de la tubulure du cathéter et de l'aiguille de tunnellisation 4.
E - Puis le praticien fait progresser l'aiguille de tunnellisation 4 sous la peau jusqu'au point d'émergence qu'il a choisi.
F - Le praticien continue alors de tirer lentement l'aiguille 4 de tunnellisation hors de la peau. Cette aiguille entraîne alors, à l'extérieur, la tubulure 3, puis le raccord 6 et les tubulures 2a et 2b de la partie intermédiaire 2, et enfin le raccord 5, ceci jusqu'à disparition totale de la boucle 15 formée lors de l'introduction de l'aiguille de tunnellisation 4 (voir figure 19).
G - A cette phase de l'opération, le praticien peut contrôler le bon positionnement du cathéter en adaptant, sur l'extrémité pointue de l'aiguille 4 de tunnellisation, un raccord spécial femelle normalisé, de préférence en polyuréthane, grâce auquel il peut contrôler le reflux de sang ou injecter du sérum hépariné dans les divers conduits, ceux quine sont pas utilisés pour l'essai étant obturés par adjonction d'une pince prévue à cet effet. Ce contrôle ayant été effectué et la dernière tubulure 3 ayant été à son tour munie d'une pince de fermeture, le praticien peut couper les deux tubes 2a et 2b dans l'ordre choisi et les munir au fur et à mesure d'un raccord femelle 16 dont l'extrémité 17 est enfoncée dans le tube correspondant.

Le praticien contrôle ensuite la fermeture du point de ponction initial et la fermeture du point d'émergence du cathéter par les pansements habituels et peut à ce moment procéder aux branchements désirés.

Ainsi, grâce à la conception particulière du présent cathéter, celui-ci peut faire l'objet d'une mise en place avec tunnellisation, bien qu'il s'agisse d'un cathéter à plusieurs lumières. Dans l'exemple décrit ci-dessus, il est prévu deux lumières dans la partie distale 1 du cathéter. Cependant celle-ci pourrait comporter trois lumières ou même plus éventuellement, auquel cas la partie intermédiaire 2 comprendrait pour sa part autant de tubes distincts raccordés chacun à l'une des lumières prévues dans la tubulure de la partie distale.

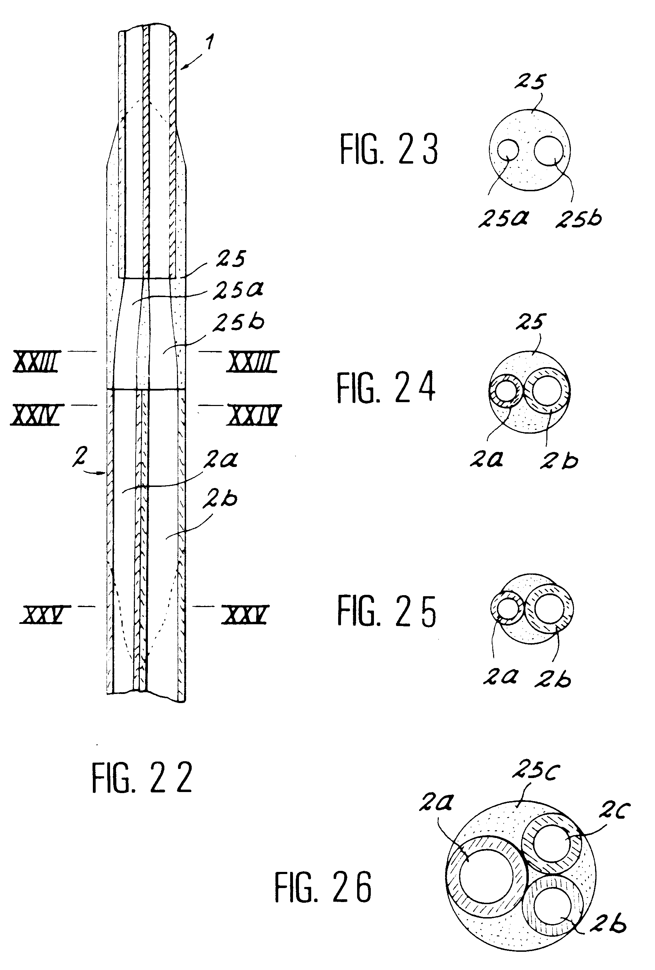

Les figures 22 à 25 illustrent une autre forme de réalisation des raccords d'un cathéter selon l'invention. Celle-ci est conçue de façon que le cathéter soit le moins traumatisant possible lors de la tunnellisation. A cet effet, le diamètre extérieur du corps de chacun des deux raccords prévus est égal au total des diamètres extérieurs des deux tubes 2a et 2b de la partie intermédiaire 2 du cathéter. Seul le premier raccord 25, qui assure la réunion de la partie distale 1 du cathéter avec sa partie intermédiaire 2, est représenté sur les figures considérées. Cependant la structure du second raccord est tout à fait similaire.

Pour éviter toute infiltration préalable de liquide entre les deux tubes 2a et 2b de la partie intermédiaire 2, et surtout pour éviter toute difficulté au moment du surmoulage du corps du raccord 25, on colle d'abord entre elles, sur une longueur d'environ 5 mm, les extrémités de ces deux tubes qui vont être incorporées dans le raccord. Dans cette forme de réalisation la section du raccord est donc réduite au minimum puisque les parois des deux tubes 2a et 2b affleurent sur les côtés, ainsi qu'il résulte de la figure 24. Dans ce cas, le corps du raccord 25 ne forme un manchon qu'autour de l'extrémité correspondante de la tubulure 1.

Bien entendu, il est avantageux d'effectuer ensuite une soudure intime des pièces, comme déjà décrit dans le cas du raccord 5 de la figure 2. A cet effet on utilise des électrodes alimentées en courant à haute fréquence et on prend les mêmes dispositions que précédemment en ce qui concerne la nature des matières plastiques des pièces en présence.

La figure 26 est une coupe transversale faite au même niveau que la figure 24 (mais à échelle différente), dans une variante 25c du raccord 25 de la figure 22, cette variante correspondant au cas où la tubulure 1 de la partie distale du cathéter comporte trois lumières. La partie intermédiaire 2 du cathéter comporte alors pour sa part trois tubes distincts 2a, 2b et 2c. Dans ce cas, la paroi externe du raccord 25a correspond au cercle circonscrit autour de ces trois tubes après avoir accolé ceux-ci de manière que le diamètre de ce cercle circonscrit soit aussi petit que possible. En conséquence, bien que dans un tel cas la partie intermédiaire 2 du cathéter comporte trois tubes distincts, la section des raccords reste très limitée, ce qui permet la tunnellisation de ce cathéter, comme déjà décrit précédemment.

Il convient enfin de noter que la section des raccords des dispositifs selon l'invention peut avoir un contour de forme autre que la forme circulaire représentée sur les dessins annexés, par exemple une forme elliptique ou ovale aussi proche que possible de la surface externe des tubulures, afin de réduire la surface totale de la section de ces raccords.

## Revendications

1. Cathéter intravasculaire comportant une tubulure multilumière destinée à la perfusion de médicaments incompatibles entre eux à l'état pur ou à la réalisation de plusieurs fonctions différentes, et dans le but de permettre l'implantation de ce cathéter avec réalisation d'une "tunnellisation", la tubulure multilumière (1) constitue seulement la partie distale de ce cathéter, qui comprend en outre :
- des tubes distincts (2a, 2b) en nombre égal aux lumières de la première tubulure et qui s'étendent en amont de celle-ci pour former la partie intermédiaire (2) apte à être passée sous la peau pour réaliser la tunnellisation,
- une tubulure monolumière (3) constituant l'extrêmité proximale de l'ensemble et qui porte en bout une aiguille rigide (4) apte à permettre la réalisation de la tunnellisation,
- deux raccords (5 et 6) assurant respectivement la jonction de chaque lumière (1a, 1b) de la tubulure multilumière (1) avec un des tubes distincts (2a ou 2b) de la partie intermédiaire (2), et le raccordement de l'extrêmité opposée de ces tubes (2a, 2b) avec l'extrêmité correspondante de la tubulure monolumière (3), caractérisé en ce que chacun de ces raccords est constitué par un corps en forme d'olive effilée, surmoulé en matière plastique sur les extrêmités des parties à réunir.

2. Cathéter selon la revendication 1, caractérisé en ce que dans chaque raccord (5,6), il existe un écart (E) entre les extrêmités des tubulures à raccorder et la partie correspondante du corps du raccord comporte des canaux (5a, 5b) ménagés à l'intérieur de la matière moulée et qui présentent un écart entre eux dans le sens transversal.

3. Cathéter selon la revendication 1 ou 2, caractérisé en ce que le corps de chaque raccord (5 ou 6) forme un manchon autour de chaque tubulure, ou chaque groupe de tubes incorporé dans l'une et l'autre extrémités de ce raccord.

4. Cathéter selon la revendication 1 ou 2, caractérisé en ce que la paroi externe du corps de chaque raccord (25, 25c) coïncide avec le cercle circonscrit autour des tubes (2a, 2b ou 2a, 2b, 2c) de la partie intermédiaire du cathéter, les parois externes de ces tubes affleurant à la surface du corps de chaque raccord.

5. Procédé de fabrication d'un cathéter intravasculaire selon la revendication 1, caractérisé en ce qu'il consiste à réunir, par surmoulage de deux raccords (5,6) en matière plastique, d'une part les extrêmités proximales de chacune des lumières (1a, 1b) d'une tubulure multilumière (1) avec un des tubes d'un ensemble de plusieurs tubes parallèles (2a, 2b), et d'autre part les extrêmités opposées de ces tubes avec l'extrêmité d'une tubulure monolumière (3) destinée à constituer la partie proximale du cathéter, des mandrins (11a, 11b) de remplissage étant au préalable enfilés dans les lumières ou les tubes à raccorder.

6. Procédé de fabrication selon la revendication 5, caractérisé en ce que lors de la mise en place des tubulures sur les mandrins (11a, 11b) de remplissage, avant surmoulage de chaque raccord, on prévoit un écart (E) dans le sens longitudinal entre les extrêmités des tubulures à raccorder, ainsi qu'un écart, dans le sens transversal, entre les mandrins (11a, 11b) dans leur partie située entre les tubulures à raccorder.

7. Procédé de fabrication selon l'une des revendications caractérisé en ce qu'après l'opération de surmoulage du corps d'un raccord (5 ou 6), on place celui-ci et les parties attenantes des tubulures correspondantes entre deux électrodes de même forme que le moule précédemment utilisé, et sur lesquelles on applique un courant à haute fréquence afin d'obtenir une soudure intime du corps du raccord avec les extrémités des tubulures.

## Claims

1. An intravascular catheter comprising a multi-channel tube intended for the perfusion of medicines which are incompatible with each other in the pure state, or to carry out a plurality of different functions and with the aim of permitting the implantation of this catheter by means of a tunneling procedure, the multi-channel tube (1) constitutes only the distal part of this catheter which further comprises:
- distinct tubes (2a, 2b) in equal number to the channels of the first tube and which extend upstream thereof to form the intermediate part (2) which is able to be passed under the skin to carry out the tunneling procedure,
- a single channel tube (3) constituting the proximal end of the assembly and which carries at the end a rigid needle (4) able to permit the tunneling procedure to be carried out,
- two connectors (5 and 6) respectively ensuring the joining of each channel (1a, 1b) of the multi-channel tube (1) with one of the distinct tubes (2a or 2b) of the intermediate part (2), and the connection of the opposite end of these tubes (2a, 2b) with the corresponding end of the single channel tube (3), characterised in that each of these connectors is comprised of a body in a tapered olive shape, duplicate moulded in plastic material on the ends of the parts to be joined.

2. A catheter according to claim 1, characterised in that in each connector (5, 6) there is a space (E) between the ends of the tubes to be connected, and the corresponding part of the body of the connector comprises channels (5a, 5b) provided inside the moulded material and which have a space between them in the transverse direction.

3. A catheter according to claim 1 or 2, characterised in that the body of each connector (5 or 6) forms a sleeve around each tube or each group of tubes incorporated in one or the other end of this connector.

4. A catheter according to claim 1 or 2, characterised in that the outer wall of the body of each connector (25, 25c) coincides with the circle defined around the tubes (2a, 2b or 2a, 2b, 2c) of the intermediate part of the catheter, the outer walls of these tubes lying flush with the surface of the body of each connector.

5. A process for producing an intravascular catheter according to claim 1, characterised in that it consists of joining, by duplicate moulding two connectors (5, 6) in plastic material, on the one hand, the proximal ends of each of the channels (1a, 1b) of a multi-channel tube (1) with one of the tubes of an assembly of a plurality of parallel tubes (2a, 2b), and on the other hand, the opposite ends of these tubes with the end of a single channel tube (3) intended to comprise the proximal part of the catheter, filling mandrels (11a, 11b) being preferably threaded into the channels or the tubes to be connected.

6. A production process according to claim 5, characterised in that when the tubes are placed on the filling mandrels (11a, 11b), prior to duplicate moulding each connector, a space (E) is provided in the longitudinal direction between the ends of the tubes to be connected, as well as a space in the transverse direction between the mandrels (11a, 11b) in their part located between the tubes to be connected.

7. A production process according to any of the claims, characterised in that after the duplicate moulding operation of the body of a connector (5 or 6) this and the adjoining parts of the corresponding tubes are placed between two electrodes which is of the same shape as the mould previously used and to which a high frequency current is applied in order to obtain an intimate soldering of the body of the connector with the ends of the tubes.

## Patentansprüche

1. Intravaskulärer Katheter mit einem mehrlumigen Rohrstutzen für die Zufuhr in reinem Zustand nicht miteinander verträglicher Medikamente oder zur Erfüllung mehrerer unterschiedlicher Funktionen, und für das Implantieren des Katheters unter "Tunnelbildung", wobei der mehrlumige Rohrstutzen (1) nur den distalen Teil des Katheters bildet und dieser darüberhinaus folgende Merkmale aufweist :
- verschiedene Rohre (2a, 2b) in gleicher Anzahl wie die Lumen des ersten Rohrstutzens, die sich unter Bildung des mittleren Teils (2) stromaufwärts erstrecken, der unter der Haut zur Tunnelbildung durchtritt,
- einen einlumigen Rohrstutzen (3), der das proximale Ende der Einheit bildet und der am Ende eine feste Punktionsnadel (4) trägt, die zur Tunnelbildung geeignet ist,
- zwei Verbindungsstücke (5 und 6), die die Verbindung jedes Lumens (1a, 1b) des mehrlumigen Rohrstutzens (1) mit den verschiedenen Rohren (2a oder 2b) des mittleren Teils (2) und den Anschluß der entgegengesetzten Enden der Rohre (2a, 2b) mit den betreffenden Enden des einlumigen Rohrstutzens (3) sicherstellen,
**dadurch gekennzeichnet**, daß jedes Verbindungsstück von einem Körper in Form einer sich verjüngenden Olive gebildet wird, die als Kunststoffmaterial auf die Enden der miteinander zu verbindenden Teile auf gegossen ist.

2. Katheter nach Anspruch 1, **dadurch gekennzeichnet**, daß in jedem Verbindungsstück (5, 6) ein Abstand (E) zwischen den Enden der zu verbindenden Rohrstutzen und daß der betreffende Teil des Körpers des Verbindungsstücks Kanäle (5a, 5b) aufweist, die im Innern des gegossenen Materials angeordnet sind und die zwischen sich einen Abstand in Querrichtung aufweisen.

3. Katheter nach Anspruch 1 oder 2**, dadurch gekennzeichnet**, daß der Körper jedes Verbindungsstücks (5 oder 6) eine Muffe um jeden Rohrstutzen oder jede Gruppe von Rohren bildet, die am einen und anderen Ende des Verbindungsstücks eingeschlossen sind.

4. Katheter nach Anspruch 1 oder 2, **dadurch gekennzeichnet**, daß die äußere Wand des Körpers jedes Verbindungsstücks (25, 25c) mit dem Umkreis um die Rohre (2a, 2b oder 2a, 2b, 2c) des mittleren Teils des Katheters übereinstimmt, wobei die äußeren Wände dieser Rohre mit der Oberfläche des Körpers jedes Verbindungsstücks fluchten.

5. Verfahren zur Herstellung eines intravaskulären Katheters nach Anspruch 1, **dadurch gekennzeichnet**, daß er zur Verbindung einerseits der proximalen Enden jedes Lumens (1a, 1b) des mehrlumigen Rohrstutzens (1) mit einem der Rohre einer Einheit aus mehreren parallelen Rohren (2a, 2b) und andererseits der anderen Enden der Rohre mit dem Ende eines einlumigen Rohrstutzens (3), der den proximalen Teil des Katheters bildet, durch Aufgießen von zwei Verbindungsstücken (5, 6) aus Kunststoff Mandrine (11a, 11b) zum Ausfüllen aufweist, die vorher in die miteinander zu verbindenden Lumen oder Rohrstutzen eingesteckt werden.

6. Verfahren nach Anspruch 5, **dadurch gekennzeichnet**, daß während des Aufschiebens der Rohrstutzen auf die zum Ausfüllen dienenden Mandrine (11a, 11b) vor dem Aufgießen jedes Verbindungsstücks ein Abstand (E) in Längsrichtung zwischen den Enden der miteinander zu verbindenden Rohrstutzen und ebenso in Querrichtung ein Abstand zwischen den Mandrinen (11a, 11b) hinsichtlich ihres Teils zwischen den zu verbindenden Rohrstutzen eingehalten wird.

7. Verfahren nach einem der Ansprüche, **dadurch gekennzeichnet**, daß nach dem Aufgießen des Körpers eines Verbindungsstücks (5 oder 6) das Verbindungsstück und seine angrenzenden Teile der betreffenden Rohrstutzen zwischen zwei Elektroden in Form der vorher benutzten Gießform gebracht werden, und daß man auf sie einen Hochfrequenzstrom anwendet, um eine innige Verschweißung des Körpers des Verbindungsstücks mit den Enden der Rohrstutzen zu erreichen.
